# EUROPÄISCHE PATENTSCHRIFT

(11) **EP 1 189 856 B1**
(45) Veröffentlichungstag und Bekanntmachung des Hinweises auf die Patenterteilung: **07.12.2005**
(21) Anmeldenummer: 00947799.3
(22) Anmeldetag: 14.06.2000
(51) Int. Cl.: C07C 17/04, C07C 19/01, C07C 17/10, C07C 19/12, C07C 17/395, C07C 19/08

(54) **UV-AKTIVIERTE CHLORIERUNG**
UV-ACTIVATED CHLORINATION
CHLORATION ACTIVEE PAR LES RAYONS ULTRAVIOLET

(30) Priorität: 16.06.1999 DE 19927394
(43) Veröffentlichungstag der Anmeldung: 27.03.2002
(73) Patentinhaber: Solvay Fluor GmbH, 30173 Hannover (DE)
(72) Erfinder: BRAUN, Max, D-30900 Wedemark (DE); EICHHOLZ, Kerstin, D-30855 Langenhagen (DE); PALSHERM, Stefan, D-30890 Barsinghausen (DE); BROSCH, Carsten, D-30173 Hannover (DE)
(74) Vertreter: Fischer, Reiner
(86) Internationale Anmeldenummer: PCT/DE2000/001953
(87) Internationale Veröffentlichungsnummer: WO 2000/076945

(56) Entgegenhaltungen:
- EP-A- 0 407 989
- WO-A-93/12058
- WO-A-97/37955
- GB-A- 2 318 350
- US-A- 4 060 469
- US-A- 5 421 971
- US-A- 5 944 962
- DATABASE WPI Section Ch, Week 199719 Derwent Publications Ltd., London, GB; Class E16, AN 1997-203978 XP002154532 & CN 1 097 189 A (ZHEJIANG PROV CHEM ACAD) , 11. Januar 1995 (1995-01-11)
- K.L. MÜLLER ET AL: "Die photochemischen Chlorierungen von cis-Dichloräthylen zu Tetrachloräthan und von Trichloräthylen zu Pentachloräthan" Z.PHYS.CHEM.ABT.B, Bd. 35, 1937, Seiten 455-462, XP000972376

## Beschreibung

Die Erfindung bezieht sich auf ein Verfahren zur Herstellung von gereinigtem 1,1,1,3,3-Pentafluorbutan durch UV-Licht unterstützte Chlorierung.

Es ist bereits seit langem bekannt, daß elementares Chlor unter Lichteinstrahlung an ungesättigte Kohlenstoffbindungen anlagert bzw. ein Austausch von Wasserstoff gegen Chlor erfolgt.

Aufgabe der vorliegenden Erfindung ist es, ein Verfahren anzugeben, mit welchem sich mit hoher Reaktionsgeschwindigkeit und hoher Selektivität gereinigtes 1,1,1,3,3-Pentafluorbutan herstellen läßt. Diese Aufgabe wird durch das Verfahren der vorliegenden Erfindung gelöst.

Das erfindungsgemäße Verfahren zur Herstellung von gereinigtem 1,1,1,3,3-Pentafluorbutan aus 1,1,1,3,3-Pentafluorbutan, das durch Verbindungen mit C-C-Doppelbindungen oder C-C-Dreifachbindungen verunreinigt ist, unter Chlorierung dieser ungesättigten Verbindungen sieht vor, dass man die Ausgangsverbindung mit elementarem Chlor unter Einstrahlung von UV-Licht einer Wellenlänge von λ ≥280 nm kontaktiert.

Dabei kann man in der Flüssigphase arbeiten oder auch in der Gasphase.

Generell kann man bei einer Temperatur im Bereich von Raumtemperatur bis 200 °C und bei einem Druck von 1 bis 10 bar (abs.) arbeiten. Die Reaktions temperatur und der Druck wird so gewählt, dass die zu behandelnde Ausgangsverbindung oder das Ausgangsgemisch in der Gasphase bzw. in der Flüssigphase vorliegt.

Das Molverhältnis von Ausgangsverbindung zu elementarem Chlor liegt bei der Anlagerung von Chlor im Bereich von 1:0,1 bis 1:10, beim Austausch von Wasserstoff gegen Chlor im Bereich von 1:0,01 bis 1:5. Will man beim Austausch von Wasserstoff gegen Chlor nur eins von zwei H-Atomen austauschen, liegt das Verhältnis von Ausgangsverbindungen zu Chlor im oberen Bereich (geringerer Chlorgehalt). Bevorzugt setzt man das Chlor in der 0,9-fachen bis 1,3-fachen Menge der stöchiometrisch benötigten Menge ein.

Die Erfindung betrifft die Reinigung von 1,1,1,3,3-Pentafluorbutan (HFC-365mfc) mit dem Ziel der Abtrennung photochlorierbarer olefinischer Verunreinigungen. Es hat sich gezeigt, dass die olefinischen Verbindungen, die herstellungsbedingt enthalten sind, im wesentlichen selektiv durch die erfindungsgemäße Photochlorierung umgewandelt und in Form der Chlorierungsprodukte vereinfacht abgetrennt werden können.

Zur Bestrahlung kann man vorteilhaft Bestrahlungslampen (z. B. Philips-Leuchtstoff-Röhren) verwenden, die nur (UV)-Licht einer Wellenlänge bei oder oberhalb von 280 nm (λ ≥280 nm) abstrahlen. Hier ist die Bestrahlung durch Quarzglas möglich. Einzige Voraussetzung für diese Variante ist, dass diese Lampen im Absorptionsbereich des elementaren Chlors emittieren. Alternativ dazu kann man auch beispielsweise Bestrahlungslampen (z. B. Hg-Mittel- oder Hochdruckstrahler) verwenden, die auch einige Linien im Bereich unterhalb von 280 nm (λ >280 nm) emittieren. Bei dieser Variante muß durch ein Glas bestrahlt werden, das nur für Licht einer Wellenlänge von 280 nm oder darüber (λ >280 nm) durchlässig ist, also den kürzerwelligen Bestrahlungsanteil mit λ <280 nm herausfiltert. Gut geeignet dafür sind beispielsweise Borosilikat-Gläser. Derartige Gläser enthalten üblicherweise 7 bis 13 % B₂O₃, 70 bis 80 % SiO₂, ferner 2 bis 7 % Al₂O₃ und 4 bis 8 % Na₂O + K₂O sowie 0 bis 5 % Erdalkalimetalloxide. Bekannte Warenzeichen für Borosilikat-Gläser sind Duran, Pyrex und Solidex. Selbstverständlich kann man auch so vorgehen, daß man einerseits eine Bestrahlungslampe einsetzt, die Licht oberhalb der angegebenen Wellenlänge abstrahlt, und zusätzlich Gläser verwendet, die für Licht oberhalb der angegebenen Wellenlänge durchlässig sind (d. h. für Licht unterhalb der angegebenen Wellenlänge entsprechend undurchlässig sind).

Gut geeignet zur Bestrahlung sind auch Lampen, z. B. Hg-Hochdrucklampen, die aufgrund eines Dotierungsmittels überwiegend oder nur im Wellenbereich bei oder oberhalb von 280 nm abstrahlen. Hg-Hochdruckstrahler beispielsweise weisen eine recht intensive Bande im Bereich von 254 nm auf, die, wie oben beschrieben worden ist, z. B. durch Borosilikat-Glas herausgefiltert wird. Bei durch Metalliodide dotierten Hg-Hochdruckstrahlern ist diese Linie stark unterdrückt. Überraschend ist die oft überproportionale Erhöhung der Umsatzrate bei solchen dotierten Strahlern. Hervorragende Ergebnisse im Hinblick auf Umsatzrate und Selektivität erzielt man mit Hg-Hochdruckstrahlern, die mit Galliumiodid dotiert sind, und besonders mit Strahlern, die mit Thalliumiodid oder Cadmiumiodid dotiert sind. Auch bei Verwendung solcher Strahler setzt man vorteilhäft Glas ein, das den kürzerwelligen Strahlungsanteil mit λ ≤280 nm herausfiltert. Zweckmäßig und technisch vorteilhaft ist es, den gesamten Strahlungsbereich mit Wellenlängen oberhalb der genannten Grenze auszunutzen.

Die Reinigung von HFC-365mfc kann in der Flüssig- oder Gasphase erfolgen. In der Gasphase ist kontinuierliches Arbeiten besonders gut möglich.

In der Gasphase führt man das Verfahren vorteilhaft in einer Durchflußapparatur durch. Man geht so vor, dass man kontinuierlich Ausgangsmaterial (die entsprechende Wasserstoff und Halogen enthaltende Ausgangsverbindung sowie Chlor) in die Durchflußapparatur einspeist und entsprechend der eingespeisten Menge kontinuierlich Reaktionsprodukt abzieht.

Die durchschnittliche Verweilzeit im Reaktionsgefäß liegt vorzugsweise zwischen 0,01 und 30 Minuten, vorzugsweise zwischen 0,01 und 3 Minuten, insbesondere zwischen 0,5 und 3,0 Minuten. Selbst bei sehr kurzen Verweilzeiten, z. B. zwischen 0,04 und 0,5 Minuten, werden schon gute Ergebnisse erzielt. Die optimale durchschnittliche Verweilzeit, die u. a. von der Lampenleistung und von geometrischen Parametern der Bestrahlungsapparatur (Durchflußapparatur) abhängig ist, kann man durch einfache Handversuche und Analyse des Produktstromes, z. B. durch Gaschromatographie, ermitteln.

Bessere Umsatzraten und höhere Selektivität können erzielt werden, wenn statt einer einzelnen Bestrahlungslampe mit bestimmter Leistung zwei oder mehr leistungsschwächere Lampen gleicher Gesamtleistung in hintereinandergeschalteten Reaktoren eingesetzt werden. Dabei wird das Produkt zweckmäßig nach Verlassen der jeweiligen Reaktionen abgetrennt, z. B. durch Ausfrieren. Auch ist eine gute Verwirbelung der Reaktionsmischung, z. B. durch geeignete Einbauten im Reaktor, oft von Vorteil. In der Flüssigphase arbeitet man bevorzugt batchweise.

Vorteil des Verfahrens ist hoher Umsatz bei hoher Selektivität.

Die folgenden Beispiele sollen die Erfindung weiter erläutern, ohne sie in ihrem Umfang einzuschränken.

### Beispiel 1:

### Entfernung von olefinischen Nebenprodukten aus 1,1,1,3,3-Pentafluorbutan (365mfc) durch Photochlorierung mit λ >280 nm

### a) Laborversuche

In 2 100 ml Glaskölbchen aus Duran® 50 werden jeweils 50 g 365mfc verunreinigt mit 7.000 ppm C₄ClF₃H₄ (2 Isomere) gegeben und gerührt.

### Thermischer Versuch:

Das eine Kölbchen wurde sofort nach der Zugabe von 0,4 g (5,6 mmol) Chlor mit Aluminiumfolie umwickelt. Nach 24 h wurde die Probe gaschromatographisch untersucht. Von den 7.000 ppm C₄ClF₃H₄ (2 Isomere) wurden noch 4450 ppm gefunden, der 365 mfc Gehalt hatte sich jedoch weit über 1 % verringert.

### Photochemischer Versuch:

Das 2. Kölbchen wurde nach der Zugabe von 0,2 g (2,8 mmol) Chlor mit einer Leuchtstofflampe von Philips (Philips-Reflektorlampen Nr. 1099415, Leistung 40 W) über Nacht bestrahlt. Anschließend wurde die Probe gaschromatographisch untersucht. Von den 7.000 ppm C₄ClF₃H₄ (2 Isomere) wurden noch 160 ppm gefunden, der 365 mfc Gehalt hatte sich nahezu nicht verändert. Nochmalige Zugabe von 0,2 g (2,8 mmol) Chlor und nochmalige Bestrahlung über Nacht ergab eine nicht mehr nachweisbare Menge an C₄ClF₃H₄ (<0,1 ppm, SIM-Lauf, GC-MSD) bei wiederum nahezu konstantem 365mfc Gehalt.

### b) Technikumsversuch

| | |
|---|---|
| Versuchsaufbau | Pfaudler-Reaktor (V = 100 1) mit aufgesetzter Glaskolonne mit Kopfkühler (Wasserkühlung). In den Deckel des Pfaudlers ist eine Hg-Tauchlampe TQ 718 von Heraeus Noblelight mit einem Tauchrohr aus Duran 50 Glas installiert. Die Bestrahlung erfolgte somit mit einer Wellenlänge λ >280 nm. Die Leistung wurde auf 700 W eingestellt. |
| Versuchsdurchführung | Das 365mfc wird in den Pfaudler gepumpt. Eine halbe Stunde vor der Chlor Dosierung wurde unter Rühren die Hg-Tauchlampe (700 Watt) eingeschaltet. Es werden durch ein Tauchrohr mit ca. 20 1/h Chlor dosiert, bis im Sim-Lauf des GC-MSD keine Olefine mehr nachweisbar waren. Nach Beendigung der Chlorierung wurde die Hg-Tauchlampe noch eine Stunde betrieben. Das so behandelte 365mfc wird abgelassen und in einer Destillationskolonne (Höhe: 3 m, Durchmesser 100 mm, gefüllt mit 10 mm Raschig Füllkörper aus Glas) feindestilliert. |

### 1.1. Versuch:

| | |
|---|---|
| 62,3 kg Edukt mit 40,9 g Chlor behandelt/Versuchsdauer 3 h/GC-Analyse Edukt (vor Photochlorierung) | 99,5 w/w % 365mfc, |
| Summe C₄ClF₃H₄ : | 0,112 w/w % |
| GC-Analyse Produkt (nachPhotochlorierung) | 99,4 w/w % 365mfc, |
| Summe C₄ClF₃H₄ | <10 ppm |

### 1.2. Versuch:

| | |
|---|---|
| 62,0 kg Edukt mit 110,9 g Chlor behandelt/Versuchsdauer 5 h/Analyse Edukt | 99,7 % 365mfc, |
| Summe C₄ClF₃H₄ | 0,210 % |
| Analyse Produkt | 99,6 w/w % 365mfc |
| Summe C₄ClF₃H₄: | <10 ppm |

| | |
|---|---|
| Reinigung | Die aus den Versuchen erhaltenen Fraktionen wurden vereinigt und in der Glaskolonne feindestilliert. Die Reinheit nach Destillation betrug 99,98 % w/w % 365 mfc. |

## Patentansprüche

1. Verfahren zur Herstellung von gereinigtem 1,1,1,3-Pentafluorbutan aus 1,1,1,3,3-Pentafluorbutan, das durch Verbindungen mit C-C-Doppelbindungen oder C-C-Dreifachbindungen verunreinigt ist unter Chlorierung dieser ungesättigten Verbindungen, wobei man die Ausgangsverbindung in der Gasphase oder Flüssigphase mit elementarem Chlor unter Einstrahlung von UV-Licht einer Wellenlänge von λ ≥280 nm kontaktiert.

2. Verfahren nach Anspruch 1, **dadurch gekennzeichnet, dass** man in der Flüssigphase arbeitet.

3. Verfahren nach Anspruch 1, **dadurch gekennzeichnet, dass** man bei einer Temperatur im Bereich von Raumtemperatur bis 200 °C arbeitet.

4. Verfahren nach Anspruch 1, **dadurch gekennzeichnet, dass** man bei einem Druck von 1 bis 10 bar (abs.) arbeitet.

5. Verfahren nach Anspruch 1 zur Reinigung von 1,1,1,3,3-Pentafluorbutan, wobei man ungesättigte Verunreinigungen in Chlor enthaltende Verunreinigungen überführt und abtrennt.

6. Verfahren nach Anspruch 1, **dadurch gekennzeichnet, dass** das elementare Chlor in der 0,9-fachen bis 1,3-fachen Menge der stöchiometrisch benötigten Menge eingesetzt wird.

7. Verfahren nach Anspruch 1, **dadurch gekennzeichnet, dass** die Verweilzeit im Reaktionsgefäß 0,01 bis 30 Minuten beträgt.

8. Verfahren nach Anspruch 1, **dadurch gekennzeichnet, dass** man Bestrahlungslampen verwendet, die nur UV-Licht bei oder oberhalb von 280 nm abstrahlen.

9. Verfahren nach Anspruch 1, **dadurch gekennzeichnet, dass** man durch ein Glas bestrahlt das den kürzerwelligen Bestrahlungsanteil, mit λ < 280 nm herausfiltert.

10. Verfahren nach Anspruch 1, **dadurch gekennzeichnet, dass** die Verunreinigungen herstellungsbedingt im 1,1,1,3,3-Pentafluorbutan enthalten sind.

## Claims

1. A process for the preparation of purified 1,1,1,3,3-pentafluorobutane from 1,1,1,3,3-pentafluorobutane which is contaminated by compounds with C-C-double bonds or C-C-triple bonds, by chlorination of these unsaturated compounds, wherein the starting compound is brought into contact in the gas phase or liquid phase with elemental chlorine with irradiation of UV light of a wavelength of λ ≥ 280 nm.

2. A process according to Claim 1, **characterised in that** operation is in the liquid phase.

3. A process according to Claim 1, **characterised in that** operation is at a temperature in the range from room temperature to 200°C.

4. A process according to Claim 1, **characterised in that** operation is at a pressure of 1 to 10 bar (absolute).

5. A process according to Claim 1 for the purification of 1,1,1,3,3-pentafluorobutane, wherein unsaturated impurities are converted into chlorinecontaining impurities and separated.

6. A process according to Claim 1, **characterised in that** the elemental chlorine is used in 0.9 to 1.3 times the stoichiometrically required amount.

7. A process according to Claim 1, **characterised in that** the residence time in the reaction vessel is 0.01 to 30 minutes.

8. A process according to Claim 1, **characterised in that** irradiation lamps which only emit UV light at or above 280 nm are used.

9. A process according to Claim 1, **characterised by** irradiation through a glass which filters out the shorter-wave portion of the radiation of λ < 280 nm.

10. A process according to Claim 1, **characterised in that** the impurities are contained in the 1,1,1,3,3-pentafluorobutane are production-related.

## Revendications

1. Procédé pour la fabrication de 1,1,1,3,3-pentafluorobutane épuré à partir de 1,1,1,3,3-pentafluorobutane, souillé par des composés ayant des doubles liaisons C-C ou des triples liaisons C-C, par chloration de ces composés insaturés, dans lequel on met en contact le composé de départ en phase gazeuse ou en phase liquide avec du chlore élémentaire sous un rayonnement UV d'une longueur d'onde λ ≥ 280 nm.

2. Procédé selon la revendication 1, **caractérisé en ce qu'**on travaille en phase liquide.

3. Procédé selon la revendication 1, **caractérisé en ce qu'**on travaille à une température située dans la plage de la température ambiante jusqu'à 200 °C.

4. Procédé selon la revendication 1, **caractérisé en ce qu'**on travaille à une pression de 1 jusqu'à 10 bars (pression absolue).

5. Procédé selon la revendication 1 pour l'épuration de 1,1,1,3,3-pentafluorobutane, dans lequel on convertit des impuretés insaturées en impuretés contenant du chlore et on les sépare.

6. Procédé selon la revendication 1, **caractérisé en ce que** du chlore élémentaire est utilisé dans une quantité de 0,9 fois jusqu'à 1,3 fois la quantité nécessaire du point de vue stoechiométrique.

7. Procédé selon la revendication 1, **caractérisé en ce que** le temps de séjour dans le réacteur est de 0,01 jusqu'à 30 minutes.

8. Procédé selon la revendication 1, **caractérisé en ce qu'**on utilise des lampes d'irradiation qui émettent seulement de la lumière UV à 280 nm ou au-dessus de cette valeur.

9. Procédé selon la revendication 1, **caractérisé en ce qu'**on irradie à travers un verre qui élimine par filtration la fraction de rayonnement de plus courte longueur d'onde λ < 280 nm.

10. Procédé selon la revendication 1, **caractérisé en ce que** les impuretés sont contenues dans le 1,1,1,3,3-pentafluorobutane en raison de la fabrication.
